# EUROPEAN PATENT SPECIFICATION

(11) **EP 1 989 313 B1**
(45) Date of publication and mention of the grant of the patent: **13.01.2016**
(21) Application number: 07713404.7
(22) Date of filing: 09.02.2007
(51) Int. Cl.: C12N 15/82, A01H 5/08, C12Q 1/68

(54) **TRANSGENIC BRINJAL (SOLANUM MELONGENA) EXPRESSING THE CRYIAC GENE**
TRANSGENE AUBERGINE (SOLANUM MELONGENA) MIT EXPRESSION DES CRYIAC-GENS
AUBERGINE (SOLANUM MELONGENA) TRANSGENIQUE EXPRIMANT LE GENE CRYIAC

(30) Priority: 10.02.2006 IN DE03682006
(43) Date of publication of application: 12.11.2008
(73) Proprietor: Maharashtra Hybrid Seeds Company Limited (MAHYCO), Paharganj, New Delhi 110 055 (IN)
(72) Inventor: CHAR, Bharat, Raghunath, Jalna 431203, Maharashtra (IN); GHANDI, Ratnapal, Popatlal, Aurangabad 431005, Maharashtra (IN)
(74) Representative: Faggioni, Marco
(86) International application number: PCT/IN2007/000050
(87) International publication number: WO 2007/091277

(56) References cited:
- WO-A-02/100163
- US-A1- 2003 024 005
- DATABASE Geneseq [Online] 14 January 1991 (1991-01-14), "Encodes full-length insecticidal protein of B.t.k. HD-73 #5." XP002440924 retrieved from EBI accession no. GSN:AAQ06561 Database accession no. AAQ06561 -& EP 0 385 962 A1 (MONSANTO CO [US] MONSANTO TECHNOLOGY LLC [US]) 5 September 1990 (1990-09-05)
- KUMAR POLUMETLA A ET AL: "Insect-resistant transgenic brinjal plants" MOLECULAR BREEDING, vol. 4, no. 1, February 1998 (1998-02), pages 33-37, XP002440922 ISSN: 1380-3743
- RANJEKAR P.K. ET AL: "Genetic engineering of crop plants for insect resistance", CURRENT SCIENCE, vol. 84, no. 3, 10 February 2003 (2003-02-10), pages 321-329,

## Description

### FIELD OF INVENTION

The present invention relates to an insect resistant transgenic brinjal plant comprising an elite event EE-1.

### BACKGROUND OF INVENTION

Brinjal has been cultivated in the country for last 4,000 years, although it is often thought of as a Mediterranean or mid-Eastern vegetable. India has been widely considered as a source of origin for brinjal. Amongst the Solanaceous vegetables, brinjal (*Solanum melongena* Linn.) is the most common, popular and principal vegetable crops grown in many geographical parts in India. The area under brinjal cultivation is estimated at 0.51 million ha. with total production of 8,200,000 Mt (FAO data, 2004, http://faostat.fao.org/). Brinjal is grown by small farmers and is an important source of income. It is a versatile crop, adapted to different agro-climatic regions and can be grown throughout the year in India. A number of cultivars are grown in the country, consumer preference being dependent upon the yield of the cultivars, fruit color, size and shape. It is a highly productive crop, the fruit are consumed as cooked vegetables in various ways, and rural people in India use dried shoots as fuel. It is a good source of minerals and vitamins and rich in total water, soluble sugars, free reducing sugars and amide proteins etc.

However, in recent years the production of brinjal in the Indian sub-continent has been seriously affected due to a steady increase in insect pest infestation, especially the fruit and shoot borer, *Leucinodes orbonalis* (Guen.). The young larvae of the fruit and shoot borer bore into petioles and midribs of large leayes and tender shoots causing shoot tips to wilt and later they bore into flower buds and fruits. The affected fruits lose their market value over and above the considerable reduction in yield. In India, it has been estimated that fruit and shoot borer causes damage to fruits ranging from 25.8 - 92.5 % and yield reduction from 20.7 - 60%.

Farmers use large quantities of chemical insecticides singly or in combination to get blemish free fruits, which fetch premium prices in the market. This practice of indiscriminate use of insecticides leads to build up of pesticide residues in the produce, destruction of natural enemies, pest resurgence and environmental pollution.

To reduce pest-linked damage in brinjal crop as well as to protect the environment from adverse effects of pesticides, deploying the lepidopteran specific cry1Ac gene under the control of a suitable promoter for high level expression in brinjal would provide an effective built-in control for fruit and shoot borer. This would result in bringing down the cultivation costs of brinjal, as contribution of chemical pesticides to brinjal cultivation is sizable.

A number of groups have carried out transformation of brinjal using different methods. The most successful of the methods are Agrobacterium-mediated methods for transformation (Kumar et. al.1998, Nicola et. al.1998, Fári et. al.1995, Rotino et. al.1990).

The source organism for *crylAc* gene is *Bacillus thuringensis* (Bt), which is a gram positive bacterium synthesizing insecticidal crystalline (Cry) inclusions during sporulation. The *crylAc* gene encodes the CrylAc protein (δ-endotoxins) of 130 kDa and is highly specific to Lepidpoteran larvae. CrylAc protein must be ingested by the insect to exhibit insecticidal activity. The protein in its crystalline form is insoluble in aqueous solution at neutral or acidic pH however the pH of the larval insect gut is alkaline which favors solubilization of the protein crystal. The solubilized protein is subsequently activated by the proteases in the insect gut. These proteases cleave the carboxy terminal domain from the rest of the protein as well as approximately 28 amino acids from the amino terminal end of the protein. The activated protein, which consists of approximately 600 amino acids, diffuses through the peritrophic membrane of the insect to the midgut epithelium. Here it binds to specific high affinity receptors on the surface of the midgut epithelium of target insects. Pores are formed in the membrane leading to leakage of intracellular content (eg. K+) into the gut lumen and water into the epithelial gut cells. The larval gut epithelial cells swell due to osmotic pressure and lyse. The gut becomes paralyzed as a consequence of changes in electrolytes and pH in the gut causing the larval insect to stop eating and die.

The expression of a foreign gene in plants is known to be influenced by the location of the transgene in the genome of the plant. Variations in transgene expression occur due to insertion into chromatin regions which may be more transcriptionally active (euchromatin) or less active (heterochromatin). Examples of these are methylated regions in which gene expression is suppressed, or in the proximity of transcriptional regulation elements like enhancers and suppressor, which increase or decrease gene expression respectively. Therefore it is necessary to screen a large number of independent transformation events for the expression of the transgene and to identify the event showing desired expression of the heterologous inserted gene.

US 2003/024005 and WO 02/100163 disclose so-called elite events, providing high and stable expression of the transgene and moreover the principle of identification of the specific event with the help of primers or the determination of the zygosity level. Kumar Polutmela A. et al "Insect-resistant transgenic brinjal plants - " Molecular Breeding , Vol. 4, no. 1, February 1998 (1998-02), pages 33-367 - discloses transgenic brinjal comprising the cry1Ab sequence.

### SUMMARY OF THE INVENTION

The present invention relates to an insect resistant transgenic brinjal plant comprising an elite event EE-1. The transgenic plant was characterized by harboring the *cry1Ac* gene under the control of CaMV 35S promoter at a specific locus in the brinjal genome. Further, the invention discloses a method for detection of the elite event EE-1 in brinjal plants. The invention further provides a kit for identification of the brinjal plants comprising the elite event EE-1.

The present invention relates to the transformation of the brinjal plant with *cry1Ac* gene for conferring insect resistance. The invention pertains to transforming brinjal plants with plant expression vector pMON10518 by *Agrobacterium-mediated* methods. Further it also relates to the production of more than 50 independent transformation events comprising the *cry1Ac* gene. All the independent events were screened and characterized for the expression of the Cry1Ac protein. Based on the level of expression of the Cry1Ac protein and insect bioassays, two events were selected for further characterization. The present invention also relates to a process of identification of transformation events. One event named as EE-1 was identified as an elite event and characterized in detail.

The specific location of the insertion of the heterologous gene was analyzed by molecular methods. This involves cloning of the genomic region flanking the right border of the T-DNA into suitable vectors. The present invention also relates to the characterization and analysis of this flanking region by sequencing. The invention relates to designing primers from this region of the DNA sequence for PCR amplification of the genomic DNA of brinjal elite EE-1 event.

The present invention further provides a diagnostic tool to distinguish the EE-1 elite event from other brinjal transformation events and non-transgenic brinjal plants.

One aspect of the present invention is to provide a method of detecting the presence of nucleic acid sequence of insect resistant brinjal plant EE-1 event comprising cry1Ac gene in a sample, wherein said method comprises:
a) contacting the sample with a first nucleotide sequence as set forth in the SEQ ID NO: 11, and a second nucleotide sequence selected from a group consisting of SEQ ID NO: 12 or SEQ ID NO: 13 or SEQ ID NO: 14.
b) performing a nucleic acid amplification reaction, thereby producing an amplified fragment; and
c) detecting said amplified fragment.

Further aspect of the invention provides an isolated polynucleotide useful for detection of insect resistant brinjal plant EE-1 event comprising *cryJAc* gene, wherein said polynucleotide comprises the polynucleotide sequence as set forth in SEQ ID NO: 8 or complement thereof.

Yet another aspect of the invention provides an isolated polynucleotide useful for detection of insect resistant brinjal plant EE-1 event comprising *cry1Ac* gene, wherein said polynucleotide comprises the polynucleotide sequence as set forth in SEQ ID NO: 9 or complement thereof.

Still another aspect of the present invention is to provide an isolated polynucleotide useful for detection of insect resistant brinjal plant EE-1 event comprising *cry1Ac* gene, wherein said polynucleotide comprises the polynucleotide sequence as set forth in SEQ ID NO: 10 or complement thereof.

Still yet another aspect of the invention is to provide a kit for detecting the presence of nucleic acid sequence of insect resistant brinjal plant EE-1 event comprising *cry1Ac* gene in a sample; wherein said kit comprises a first nucleotide having sequence as set forth in the SEQ ID NO: 11 and a second nucleotide sequence selected from a group consisting of SEQ ID NO: 12 or SEQ ID NO: 13 or SEQ ID NO: 14.

The present invention also provides a synthetic oligonucleotide set comprising two oligonucleotides for detecting the presence of insect resistant brinjal plant EE-1 event comprising *cry1Ac* gene, wherein the first oligonucleotide comprises the nucleotide sequence as set forth in SEQ ID NO: 11 and the second oligonucleotide comprises the nucleotide sequence selected from a group consisting of SEQ ID NO: 12, SEQ ID NO: 13 and SEQ ID NO: 14.

### BRIEF DESCRIPTION OF ACCOMPANYING DRAWINGS

Figure 1 shows a map of the construct pMON10518
Figure 2 shows gel image of the amplified product from brinjal plants using the event specific primers
   Lane 1: phage lambda/*Hin*d III marker
   Lane 2: DNA from the EE-1 elite event containing plant showing an amplified fragment of 580 bp
   Lane 3 and 4: DNA from transgenic brinjal plants that do not contain the EE-1 event
   Lane 5: DNA template from a non-transgenic brinjal plant
   Lane 6: no DNA (water) - negative control

### DETAILED DESCRIPTION OF THE INVENTION

The term "amplicon" or "amplified DNA" "amplified fragment" refers to the product of nucleic acid amplification of a target nucleic acid sequence that is part of nucleic acid template.

The term "Heterologous Gene/DNA" refers to DNA sequence of foreign origin inserted in the plant genome.

The term "event" refers to the original transformant and any progeny produced by a sexual outcross between the original transformant or its descendants bearing the heterologous gene, and another brinjal variety.

The term "probe" refers to a DNA sequence useful for hybridization experiments or assay.

By hybridizing under "stringent conditions" is meant the conventional hybridizing conditions as described by Sambrook et al. (1989) (Molecular Cloning: A Laboratory Manual, Second Edition, Cold Spring Harbour Laboratory Press, NY).

An "elite event", as used herein, is an event which is selected from a group of events, obtained by transformation with the same transforming DNA or by back-crossing with plants obtained by such transformation, based on the phenotypic expression and stability of the transgenes and the absence of negative impact on the agronomic characteristics of the plant comprising it (i.e., selected transformation event).

The present invention relates to an insect resistant transgenic brinjal plant comprising an elite event EE-1. The present invention relates to an insect resistant transgenic brinjal plant comprising an elite event EE-1. The transgenic plant is characterized by harboring the *cry1Ac* gene under the control of CaMV 35S promoter at a specific locus in the brinjal genome. Further, the invention discloses a method for detection of an elite event EE-1 in transgenic brinjal plant. The invention further provides a kit for identification of the transgenic plants comprising the elite event EE-1.

In one embodiment of the present invention is to provide a method of detecting the presence of nucleic acid sequence of insect resistant brinjal plant EE-1 event comprising cry1Ac gene in a sample, wherein said method comprises:
a) contacting the sample with a first nucleotide sequence as set forth in the SEQ ID NO: 11, and a second nucleotide sequence selected from a group consisting of SEQ ID NO: 12 or SEQ ID NO: 13 or SEQ ID NO: 14.
b) performing a nucleic acid amplification reaction, thereby producing an amplified fragment; and
c) detecting said amplified fragment.

Yet another embodiment of the present invention is to provide an isolated polynucleotide useful for detection of insect resistant brinjal plant EE-1 event comprising *cry1Ac* gene, wherein said polynucleotide comprises the polynucleotide sequence as set forth in SEQ ID NO: 8 or complement thereof.

Still yet another embodiment of the present invention provides an isolated polynucleotide useful for detection of insect resistant brinja1 plant EE-1 event comprising *cry1Ac* gene, wherein said polynucleotide comprises the polynucleotide sequence as set forth in SEQ ID NO: 9 or complement thereof.

Additional embodiment of the present invention is to provide an isolated polynucleotide useful for detection of insect resistant brinjal plant EE-1 event comprising *cry1Ac* gene, wherein said polynucleotide comprises the polynucleotide sequence as set forth in SEQ ID NO: 10 or complement thereof.

Further the present invention provides a kit for detecting the presence of nucleic acid sequence of insect resistant brinjal plant EE-1 event comprising *cry1Ac* gene in a sample; wherein said kit comprises a first nucleotide having sequence as set forth in the SEQ ID NO: 11 and a second nucleotide sequence selected from a group consisting of SEQ ID NO: 12 or SEQ ID NO: 13 or SEQ ID NO: 14.

The present invention also provides a synthetic oligonucleotide set comprising two oligonucleotides for detecting the presence of insect resistant brinjal plant EE-1 event comprising *cry1Ac* gene, wherein the first oligonucleotide comprises the nucleotide sequence as set forth in SEQ ID NO: 11 and the second oligonucleotide comprises the nucleotide sequence selected from a group consisting of SEQ ID NO: 12, SEQ ID NO: 13 and SEQ ID NO: 14.

Disclosed is a method for transforming plant, plant cells and tissues of Brinjal (*Solanum melongena*) using *Agrobacterium-*mediated transformation method for conferring resistance to insect pests.

Also disclosed are explants for transformation that are selected from a group consisting of cotyledon with petiole, hypocotyls, embryo, immature embryo, leaf lamina, cotyledonary axil, shoot tip, anther, root and callus or any other suitable explant.

Another embodiment of the present invention is to provide a method of identification of the flanking sequence around the transgenic insertion site for the brinjal plant elite event EE-1 by PCR amplification or methods known in the art. Nucleic acid amplification can be accomplished by any of the various nucleic acid amplification methods known in the art, including the polymerase chain reaction (PCR). The method of identification of brinjal plant elite event EE-1 was carried out by primer walking method. This can also be carried out by the method well known in the art.

Transgenic insertion and neighboring flanking brinjal DNA were purified by agarose gel electrophoresis and cloned suitable vector known in the art. The cloned fragment was sequenced by methods known in the art.

Another embodiment of the present invention is to provide diagnostic methods or assay for identification of the brinjal plant EE-1 elite event.

Further described is a method of introduction of EE-1 elite event in other background or cultivars of brinjal.

Also described is a method of production of brinjal hybrids using the transgenic brinjal having EE-1 elite event.

Yet another embodiment of the present invention provides a novel DNA molecule having polynucleotide sequence as set forth in the SEQ ID NO: 8-10 and the complement thereof.

Further embodiment of the invention provide transgenic brinjal plant, plant cell, seed and progeny comprising the polynucleotide sequence selected from the group consisting of SEQ ID NO: 8, SEQ ID NO: 9, and SEQ ID NO: 10

Also described is a method of producing a transgenic Brinjal plant resistant to insect pests comprising transforming a brinjal cell with the DNA construct pMON10518. The fertile brinjal plant obtained from the said brinjal cell can be self pollinated or crossed with compatible brinjal varieties to produce insect resistant brinjal plant.

Insecticidal *cry1Ac* gene from *Bacillus thruingiensis* has been transferred into brinjal line 60208 developed by MAHYCO. The present invention provides an efficient method for transforming plant, plant cells and tissues of brinjal (*Solanum melongena*) plant using *Agrobacterium-* mediated transformation method for conferring resistance to insect pests.

The explants for *Agrobacterium* mediated transformation of brinjal plant were selected from a group consisting of cotyledon with petiole, hypocotyls, embryo, immature embryo, leaf lamina, cotyledonary axil, shoot tip, anther, root and callus or any other suitable explant.

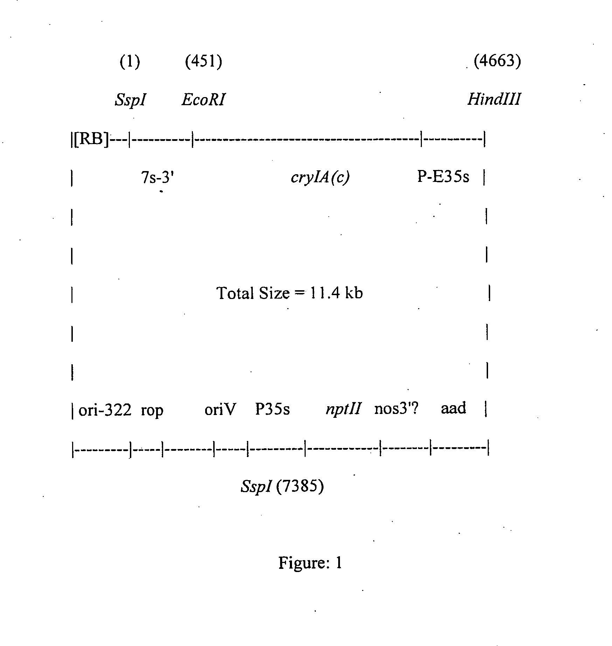

The vector pMON10518 (Figure 1) containing *cry1Ac* gene under the control of CaMV e35S promoter and Gm7S terminator; *nptII* gene under the control of CaMV 35S promoter as a plant selectable marker gene was transformed in the *Agrobacterium tumefaciens* cells. The recombinant *A. tumefaciens* was inoculated into a suitable medium for the growth of *Agrobacterium. Agrobacterium* cells were inoculated into 25 ml of sterile 2YT medium (pH 7) in a flask. 2YT medium contains 1% Yeast extract, 1.6% Tryptone and 0.5% NaCl. Suitable antibiotics were added to this medium before inoculating bacteria for the selective growth of *Agrobacterium* with the plasmid pMON10518. The bacteria were inoculated in 2YT medium in flask and kept on a shaker to get Optical Density (600nm) in the range of 0.01 to 2, preferably 1.8.

Explants were inoculated in recombinant *Agrobacterium* suspension (preferably 15 minutes), blotted dry on sterile filter paper and later transferred to petri plates containing suitable growth medium for co-cultivation.

After the co-cultivation (2 to 5 days preferably 2 days of co-cultivation), these explants were washed in liquid MS0 medium with 500mg/l Cefotaxime to inhibit the growth of *Agrobacterium* and were transferred on selection medium.

Transformants regenerated on the selection medium were transferred to rooting medium and the rooted plants were hardened and established in green house.

Detailed procedure of transformation of brinjal plant with the pMON10518 construct is provided in the Example 1.

In the present invention the *Cry1Ac* gene from *Bacillus thruingiensis* has been transferred into brinjal line 60208 developed by MAHYCO which has the following characters:

| | |
|---|---|
| Fruit color: | Purple, white variegated |
| Fruit shape: | Oval |
| Plant habit: | Bushy |
| Fruit development: | In clusters |
| Calyx: | Spiny |

Over 50 independent transformation events were screened to identify an elite brinjal plant EE-1 event. All events underwent transgene segregation analysis and protein expression evaluation to determine the optimum event for commercialization. Details are provided in the Example 2.

Molecular characterization of the brinjal plant EE-1 elite event was carried out.. Details are provided in the Example 3. Further diagnostic methods for identification of the brinjal plant EE-1 elite event was carried out details are provided in Example 4. Example 5 describes the zygotic assay carried out for brinjal plant EE-1 elite event.

Present invention further provides a method of detection of the presence of brinjal plant EE-1 elite event nucleic acid sequence in a sample using Southern hybridization method as described by Sambrook et al. (1989) (Molecular Cloning: A Laboratory Manual, Second Edition, Cold Spring Harbour Laboratory Press, NY). This method comprises 1) immobilizing plant genomic DNA fragments on a filter, 2) prehybridizing the filter for 1 to 2 hours at 42°C in 50% formamide, 5XSSPE, 2X Denhardt's reagent and 0.1% SDS, or for 1 to 2 hours at 68°C in 6X SSC, 2X Denhardt's reagent and 0.1% SDS, 3) adding the hybridization probe which has been labeled, 4) incubating for 16 to 24 hours, 5) washing the filter for 20 min. at room temperature in 1X.SSC, 0.1% SDS, 6) washing the filter three times for 20 min. each at 68°C in 0.2X SSC, 0.1% SDS, and 7) exposing the filter for 24 to 48 hours to X-ray film at -70°C with an intensifying screen. The hybridization may be carried out by methods known in the art.

The brinjal plant EE-1 elite event was chosen on the basis of a number of criteria. Segregation analysis over three generations indicated that there is a single locus of insertion of the *cry1Ac* gene in this line. This was confirmed by DNA blot analysis. Protein quantification studies using quantitative ELISA were performed on a number of brinjal single insertion events. These studies indicated that the EE-1 line was the highest Cry1Ac protein expressing line, and that the expression of the inserted gene was stable in a number of different genetic backgrounds, over multiple generations. Phenotypic analysis of the EE-1 elite event bearing line showed that it was morphologically indistinguishable from the non-transformed parent line from which it was derived, and therefore most suitable for further backcross breeding. The elite plant was employed to transfer the EE 1 elite event in other brinjal cultivars.

Nucleic acid amplification can be accomplished by any of the various nucleic acid amplification methods known in the art, including the polymerase chain reaction (PCR). A variety of amplification methods are known in the art and are described in PCR protocols: a guide to methods and applications (ed. Innis et al., Academic Press, San Diego, 1990). Transgenic insertion and neighboring flanking brinjal DNA were purified by agarose gel electrophoresis and cloned. The cloned fragment was sequenced by methods known in the art.

While the invention is broadly as defined above, it will be appreciated by those persons skilled in the art that it is not limited thereto and that it also includes embodiments of which the following description gives examples.

### EXAMPLES

The examples given are merely illustrative of the uses, processes and products claimed in this invention, and the practice of the invention itself is not restricted to or by the examples described.

### Example 1

### Transformation of Brinjal

### Sterilization and inoculation of seeds

Brinjal seeds from line 60208 were surface sterilised in a 50 ml plastic centrifuge tube with 1.5 % NaOCl for 10 min. with vigorous shaking (20 ml NaOCl for 500 seeds). After 5 min the solution was decanted arid the seeds washed 5 times with sterile distilled water. The seeds were blotted dry on sterile filter paper for 1 hr and inoculated on MS0 medium (Table 1) in bottles at 10 seeds/bottle. The seeds were maintained at 25°C for 12-15 days to germinate with a photoperiod regime of 16 hrs light and 8 hrs darkness.

### Agrobacterium cultures

A day before co-cultivation was to be done, a culture of the *Agrobacterium* strain harbouring the transformation vector pMON10518 was grown overnight in 25 ml liquid LB medium (Table 1) at 28°C with shaking at 175 rpm with the respective antibiotics. This overnight culture was started with a loopful of bacterial cells taken from a freshly-streaked solid medium plate containing the same antibiotics.

### Explant preparation

On the day of co-cultivation, cotyledons from 2-15 days old seedlings were removed and cut longitudinally in half through the midrib, and used as explants. The explants were kept in petridishes on sterile filter papers soaked in liquid MS medium (Table 1) to prevent the explants from drying out.

### Co-cultivation

On the morning of co-cultivation, the overnight grown bacterial culture was centrifuged at 10000 rpm for 10 min. The supernatant was discarded and the pellet resuspended in liquid. MS medium (25 ml) and mixed well (Table 1). 25 ul of 100mM acetosyringone was added to the bacterial culture which was then placed in the incubator for growth with shaking for a further 2 hr. The optical density (OD) of the bacterial culture was measured at 600 nm until an OD of 1.5 to 1.8 was reached. The explants were incubated in the bacterial culture in a petridish or a glass beaker for 10 min with slow stirring. The explants were then blotted on a sterile filter paper to remove excess bacteria and placed on co-cultivation medium B1AsP (Please find the media composition in table) for the three days for co-cultivation. (15 explants/ plate). The plates were incubated under light at 25°C for three days with a photoperiod regime of 16 hrs light + 8 hrs darkness.

Positive and negative controls were also maintained in each experiment. Positive controls were explants regenerated on medium without antibiotics to check the tissue culture regeneration, whereas negative controls are explants maintained on antibiotic-containing media to make sure the antibiotic is checking the growth.

### Selection (B 1 KC medium)

After 3 days the explants were transferred on selection medium B1KC (Table 1) medium with kanamycin 50 mg/l and cefatoxime 250 mg/l for a period of 2 weeks. Transformed explants produce calli at the cut end of explants, and non- transformed explants bleach. Putative transformed explants were maintained on fresh selection medium B1KC again for two weeks. This was repeated for a total period of 6 weeks on selection medium. At the end of the sixth week, green meristematic tissues/calli develops.

### Regeneration (CF2KC medium)

Green meristematic tissues were transferred onto regeneration medium, CF2 KC (Table 1), with kanamycin 50 mg/l and cefotaxime 250mg/l for a period 2 weeks. The calli were subcultured three times every 2 weeks (3 selections) on fresh medium resulting in small green shoot buds developing from the calli.

### Elongation (B root KC medium)

The shoot buds were transferred onto BrootKC medium (Table 1) with kanamycin 50 mg/lit and cefotaxime 250mg/l for a period of 2 weeks. The shoot buds were twice subcultured every 2 weeks (2 selections) on fresh medium. At the end of the fourth week, the shoot buds elongate and are ready for rooting. Sometimes on the elongation medium rooting may begin. If rooting starts on the elongation medium the plantlets were left undisturbed in order to avoid damage to the roots.

### Rooting (TMRGKC medium)

The shoots were transferred onto rooting medium TMRGKC (Table 1) with kanamycin 50 mg/l and cefotaxime 250mg/li for a period of 2 weeks. The shoots were subcultured every 2 weeks till rooting occurred.

### Hardening

The rooted plants were washed with sterile distilled water thoroughly to remove the gelling agent (agar or phytagel). The plants were treated with 0.1 % Bavistin for at least 1 hr before transferring to cups containing mixture of promix (60%) and soil (40%). The plants were covered with polythene bags for 7 days. After 7 days, the polythene bags were cut from the comers to allow the hardening process to begin, which is completed in about 2 weeks.

**Table 1: Composition of media used in brinjal transformation**

| **Sr. No.** | **Medium** | **Composition** | **Purpose** |
|---|---|---|---|
| 1 | LB Solid | Trytone 10gm/L, Yeast extract 5gm/L, NaCl 10 gm/L, pH 7.0, Agar Agar 15gm/L | Bacterial culture |
| 2 | LB liquid | Trytone 10gm/L, Yeast extract 5gm/L, NaCl 10 gm/L, pH 7.0 | Bacteria inoculation. |
| 3 | MS liquid | MS major 100ml/L, MS minor 10ml/L, MS CaCl2 10ml/L, MS Iron 10ml/L, MS Vitamins 10ml/L, 10ml/L Sucrose 3%, 5.8 | Bacteria resuspension |
| 4 | MS0 | MS major 100ml/L, MS minor 10ml/L,MS CaCl2 10ml/L, MS Iron 10ml/L, B5 Vitamines 10ml/L, Myoinositol 10ml/L, Sucrose 3%, pH 5.8, Agar Agar 0.8% | Seed inoculation |
| 5 | B1AsP | MS major 100ml/L, MS minor 10ml/L,MS CaCl2 10ml/L, MS Iron 10ml/L, B5 Vitamins 10ml/L, Myoinositol 10ml/L, NAA 0.05mg/L, Zeatine 2mg/L, Acetosyringone 100uM, Glucose 1.5%, pH | Co-cultivation |

| **Sr. No.** | **Medium** | **Medium Composition** | **Purpose** |
|---|---|---|---|
| 6 | BlKC | 5.8, Phytagel 0.35% MS major 100ml/L, MS minor 10ml/L,MS CaCl2 10ml/L, MS Iron 10ml/L, B5 Vitamins 10ml/L, Myoinositol 10ml/L, NAA 0.05mg/L, Zeatine 2mg/L, Glucose 1.5%, Kanamycin 50 mg/l, Cefotaxime 250 mg/l, pH 5.8, Agar Agar 0.8% | Selection. |
| 7 | CF2KC | MS major 100ml/L, MS minor 10ml/L,MS CaCl2 10ml/L, MS Iron 10ml/L, B5 Vitamines 10ml/L, Myoinositol 10ml/L, BAP 2.5mg/L, Kinetin 1mg/l, 1 Adenine(freebase) 2mg/L, IAA 0.5mg/l, Sucrose 3% Kanamycin 50 mg/l, Cefotaxime 250 mg/l, pH 5.8, Agar Agar 0.8% | Regeneration |
| 8 | Broot KC | MS major 100ml/L, MS minor 10ml/L,MS CaCl2 10ml/L, MS Iron 10ml/L, MS Vitamines 10ml/L, Myoinositol 10ml/L, IAA 0.5mg/l, Glucose 1.5% Kanamycin 50 mg/l, Cefotaxime 250 mg/l, pH 5.8, Agar Agar 0.8% | Elongation. |
| 9 | TMRGK C | MS major 50ml/L, MS minor 10ml/L, MS Cacl2 10ml/L, MS Iron 10ml/L, B5 Vitamins 10ml/L, Myoinositol 10ml.L, Glucose 0.75%, Kanamycin ; 50mg/L, Cefotaxime 250mg/L, pH 5.8, Phytagel 0.35%. | Rooting. |

### Example 2

### Identification of brinjal plant EE-1 elite event

A large number (>50) of independent transformation events were generated in order to maximize the chance of a high-transgene-expressing, genetically stable event for production of commercial transgenic brinjal lines. All brinjal plants coming out of the transformation experiments were analyzed for presence of the *cry1Ac* gene by PCR, and the positive plants subjected to ELISA for determining the expressivity of the transgene. The initial transformants (TO) were advanced to the next generation by selfing, and the T1 progeny plants were checked by PCR to determine the segregation of the transgene. The expected T1 segregation ratio for the transgene in a line with a single *cry1Ac* gene insertion is 3:1 based on Mendelian genetics. Further, as *cry1Ac* acts as a dominant gene when introduced as a transgene, the expression of the gene was monitored by ELISA in the T1 generation. Again, in a single insertion event, the expected ratio of Cry1Ac expressing plants to non-expressing plants is 3:1. Insect bioassays were carried out on tissue from selected lines in order to determine which lines would have better efficacy against the fruit and shoot borer pest. Southern blot analysis of selected individual transformation events was carried out to confirm the number of loci (insert copy number) at which the transgene integrated in the brinjal genome.

Based on the above criteria, transformed lines were selected which displayed segregation characteristics of single locus insertion events and showed effective tolerance to fruit and shoot borer. Conversely, those lines that were found to have abnormal segregation ratios and/or low efficacy against the pest were not taken further. The lines selected for advancement were grown in the greenhouse and Cry1Ac protein was estimated through the life of the crop by quantitative ELISA, which enables determination of the highest protein expressing lines. The tissues analyzed were leaf, shoot, stem, flower and root. After a careful analysis of the above parameters, event EE-1 was found to be the best available event, in terms of Cry1Ac expression, efficacy against the pest and genetic stability over three plant generations. The EE-1 elite event was used for further breeding for developing fruit and shoot borer tolerant brinjal.

### Example 3

### Molecular characterization of the EE-1 elite event

The brinjal transgenic EE-1 elite event was analyzed to identify brinjal genomic DNA sequences flanking the *cry1Ac* gene expression cassette using the method of Cottage et al., 2001. Plant genomic DNA was extracted from fresh young leaves of EE-1 elite event bearing plants using the method known in the art. Genomic DNA (2µg) was digested with *Xmn*I enzyme in 20 µl of reaction volume using standard buffers. The digestion reaction was incubated at 37°C overnight. The digestion product was then incubated at 65°C for enzyme inactivation and was precipitated with 3M sodium acetate and ethanol. DNA was air dried and dissolved in 12 µl sterile distilled water. Digested DNA was ligated to the annealed adapter in ligase buffer supplied by the manufacturer. The sequences of the adapters are as below
ADAP 1: 5'-CTA ATACGACTCACTATAGGGCGGCCGCCCGGGCAGGT- 3'SEQ ID. NO: 1
ADAP 2: 5'- P-ACC TGC CC-H₂N -3' SEQ ID NO: 2

Both the adapters were at first annealed to each other and then ligated to the digested genomic DNA of EE-1 elite event.

The ligation mixture was incubated at 15-16°C overnight for ligation of digested genomic DNA to the annealed adapters. The ligation mixture was diluted to 100 µl for obtaining adapter library, and first round amplification was carried out using the following primer combination: Forward primer complementary to the inserted heterologous DNA SEQ ID NO: 3 , and reverse primer complementary to the adapter DNA sequence SEQ ID NO: 4.
MHIP-10 - 5'- TTG GGT CTT TCA GAC TGA CAA G -3' SEQ ID NO: 3
AP - 5'- GGA TCC TAA TAC GAC TCA CTA TAG GGA-3' SEQ ID NO: 4

### Restriction digestion:

| | |
|---|---|
| Genomic DNA | 12.0 µl (2 µg) |
| 10X Reaction buffer | 2.0 µl (final concentration 1x) |
| *Xmn*I enzyme | 1.0 µl (10units/ µl) |
| Sterile water | Make up the volume to 20.0 µl. |

### Ligation:

| | |
|---|---|
| Digested Genomic DNA (heat inactivated) | 12.0 µl (2 µg) |
| Annealed adapters | 2.0 µl (100 ng/µl) |
| 10X Reaction buffer | 3.0 µl (final concentration 1x) |
| T4-ligase enzyme | 1.0 µl (5units/ µl) |
| Sterile water | Make up the volume to 30.0 µl |

### First PCR:

| **Reagents** | **Volume** |
|---|---|
| Nuclease-free water | Make up to 25 µl |
| 10X reaction buffer (with MgCl₂) | 2.5 µl |
| 10mM dNTPs | 0.5 µl |
| Primer MHIP-10(100ng/µl) | 1.0 µl |
| Primer AP (100 ng/µl) | 1.0 µl |
| Taq DNA polymerase (5 units/µl) | 0.5 µl |
| DNA template | 3.0 µl |

### Thermal Cycler program:

| **Temperature** | **Time** | **Cycles** |
|---|---|---|
| 95°C | 5 minutes | 1 |
| 94°C | 30 seconds | |
| 58°C | 30 seconds | 40 |
| 68°C | 4 minutes | |
| 68°C | 10 minutes | 1 |
| 4°C | Hold | |

The second round of PCR was necessary to obtain the specific flanking region adjacent to the inserted heterologous gene. PCR was carried out with forward primer (SEQ ID NO: 5) and a reverse primer (SEQ ID NO: 6). Details are given below:
MHIP-11- 5'- CTG ACA AGA TCG ATC TGA AGT C -3' SEQ ID NO: 5
NAP - 5'- TAT AGG GCT CGA GCG GC-3' SEQ ID NO: 6

### Second PCR:

| **Reagents** | **Volume** |
|---|---|
| Nuclease-free water | Make up to 25 µl |
| 10X reaction buffer (with | 2.5 µl |
| MgCl₂) | |
| 10mM dNTPs | 0.5 µl |
| Primer MHIP-11(100ng/µl) | 1.0 µl |
| Primer NAP (100 ng/µl) | 1.0 µl |
| Taq DNA polymerase (5 | 0.5 µl |
| units/µl) | |
| DNA template | 2.0 µl |

### Thermal Cycler program:

| **Temperature** | **Time** | **Cycles** |
|---|---|---|
| 95°C | 5 minutes | 1 |
| 94°C | 30 seconds | |
| 58°C | 30 seconds | 40 |
| 68°C | 4 minutes | |
| 68°C | 10 minutes | 1 |
| 4°C | Hold | |

A small amount of PCR product was analyzed on a 1% agarose gel, and the amplified fragment was eluted from the gel by using the method known in the art. A DNA fragment (amplicon) of 309 bp was amplified from the right border region of the T-DNA after two rounds of PCR (using primers MHIP-11 SEQ ID NO: 5 and NAP SEQ ID NO: 6). The amplified fragment (amplicon) was cloned into pGEM-T Easy vector to obtain a recombinant vector. This recombinant vector was transformed in the strain of *E. coli* by using method known in the art. The strains can be DH5α, Top 10 etc. The clone comprising this recombinant vector selected for analyzing the sequence was designated as EE-1-XmnI-4. Plasmid DNA from the clone EE-1-XmnI-4 was isolated using standard methods known in the art. The cloned fragment (amplicon) was sequenced using T7 primer. The polynucleotide sequence obtained is as shown in SEQ ID NO: 7. The sequence contains the T-DNA vector sequence consisting of a part of the *Gm7S* terminator and right border, the adapter sequence and EE-1 T-DNA flanking brinjal genomic DNA sequence.

Sequence ID NO: 7 consists of a part of the T-DNA sequence starting with primer MHIP-11 (SEQ ID NO: 5, base pair 1-22) followed by right border sequence (base pairs 97-142) adjacent to which is flanking brinjal genomic DNA sequence of EE-1 elite event (base pairs 143 to 278). The flanking genomic sequence is followed by adapter sequence (base pairs 279 to 309).
SEQ ID NO: 7

CTGACAAGATCGATCTGAAGTCTAAACAATTCTAAGAGGTATCATGTAGCAGTGTCC
TGCCACAATATTGAATTGACCTGCAGGCATGCAAGCTGGATCAGATAGTCGTTTCC
CGCCTTCAGTTTAAACTATCAGTGTTTGTTACCAAAAGTGCTGTCAATAAACACTTA
GAAAGGTGTTATCCAAATTTTAAAAAGAAAAAAATAAATGTAAGATTTAAATTTCTA
ACTTCAAGAGATGCATTCAATTATCACCGCATTATATGATATTCCAAGAATACCTGC
CCGGGCGGCCGCTCGAGCCCTATA

Sequence ID NO: 8 consists of right border sequence (1-46) followed by the flanking brinjal genomic DNA sequence of EE-1 elite event (47-182). The complete polynucleotide sequence is shown in the SEQ ID NO: 8.
SEQ ID NO: 8

GATCAGATAGTCGTTTCCCGCCTTCAGTTTAAACTATCAGTGTTTGTTACCAAAGT
GCTGTCAATAAACACTTAGAAAGGTGTTATCCAAATTTTAAAAGAAAAAAATAAAT
GTAAGATTTAAATTTCTAACTTCAAGAGATGCATTCAATTATCACCGCATTATATGA
TATTCCAAGAA

Sequence ID NO: 9 consists of right border sequence (1-41) followed by the flanking brinjal genomic DNA sequence of EE-1 elite event (41-125). The complete polynucleotide sequence is shown in the SEQ ID NO: 8.
SEQ ID NO: 9

GATCAGATAGTCGTTTCCCGCCTTCAGTTTAAACTATCAGTGTTTGTTACCAAAAGT
GCTGTCAATAAACACTTAGAAAGGTGTTATCCAAATTTTAAAAAGAAAAAAATAAAT
GTAAGATTTAA

Sequence ID NO: 10 consists of right border sequence (1-15) followed by the flanking brinjal genomic DNA sequence of EE-1 elite event (16-75). The complete polynucleotide sequence is shown in the SEQ ID NO: 10.
SEQ ID NO: 10

GTTTAAACTATCAGTGTTTGTTACCAAAAGTGCTGTCAATAAACACTTAGAAAGGTG
TTATCCAAATTTTAAAAA

### Example 4

### Diagnostic methods for identification of the EE-1 elite event

### PCR method

To detect the presence or absence of the brinjal EE-1 elite event, a molecular diagnostic method was developed. The sequence analysis of the fragment shown as SEQ ID NO: 8 was carried out and the primers were designed to amplify the transgenic insertion locus for use as a diagnostic tool. The two primers designed were forward primer MHTBJ-2 (SEQ ID NO: 11) and the second primer is MHIP-2 (SEQ ID NO: 12) to amplify the transgenic insertion locus from EE-1 genomic DNA.
MHTBJ-2: 5'- TGC GGT GAT AAT TGA ATG CAT C -3' SEQ ID NO: 11
MHIP-2: 5'- GGA GCT TCT CTT GAT GGA GG -3' SEQ ID NO: 12

These primer pairs include, but are not limited to, SEQ ID NO: 11 and SEQ ID NO: 12. For the amplification of the 3'region, any primer pair derived from SEQ ID NO: 8 that when used in DNA amplification reaction produces a DNA amplicon diagnostic for EE-1 elite event is an aspect of the present invention. Polynucleotide sequence from *Cry1Ac* gene to right border (from nucleotide position 1 to 4091 positions provided in the SEQ ID NO: 16). The complete polynucleotide sequence consisting of *cry1Ac* gene flanked by GM7S terminator followed by right border which is further followed by brinjal genomic DNA and adopter sequence is provided in SEQ ID NO: 16.

However, any modification of these methods that use DNA molecules or complements thereof to produce an amplicon DNA molecule diagnostic for EE-1 is within the ordinary skill of the art. For example if SEQ ID: 11 primer used in combination with primer1 (SEQ ID NO: 13) will produce an amplicon of 1012 base pair, or in combination with primer 2 (SEQ ID NO: 14) will amplify 1211 base pair from EE-1 event. The sequences of primer 1 and 2 are as below.
Primer 1:- 5'- CGAGCTTAAGTTCTCCAACTGC-3' SEQ ID NO: 13
Primer 2:- 5'- GGAATGTGAAAGGTCATGTGG- 3' SEQ ID NO: 14

For the analysis it is important to have positive and negative controls. The PCR method was designed in order to distinguish the EE-1 elite event from the other brinjal transgenic events and non-transgenic lines. Genomic DNA from Brinjal EE-1 elite event was isolated from leaves using the method known in the art. Genomic DNA was also isolated from other brinjal transgenic events and non-transgenic brinjal lines as controls for the PCR detection method. A control reaction having no DNA in the reaction mixture was also included.

The genomic DNA from different plants was subjected to amplification using two primers namely SEQ ID NO: 11 and SEQ ID NO: 12, the details are as follows:

| **Reagents** | **Amount to be added** |
|---|---|
| Nuclease-free water | Make up to 25 µl |
| 10X reaction buffer (with MgCl₂) | 2.5 µl |
| 10mM dNTPs | 0.5 µl |
| Primer MHIP-2 (100 ng/µl) | 1.0 µl |
| Primer MHTBJ-2 (100 ng/µl) | 1.0 µl |
| Taq DNA polymerase (5 units/µl) | 0.5 µl |
| DNA template | 2.0 µl |

| **Temperature** | **Time** | **Cycles** |
|---|---|---|
| 95°C | 5 minutes | 1 |
| 94°C | 30 seconds | |
| 58°C | 30 seconds | 35 |
| 72°C | 1 minute | |
| 72°C | 5 minutes | 1 |
| 4°C | Hold | --- |

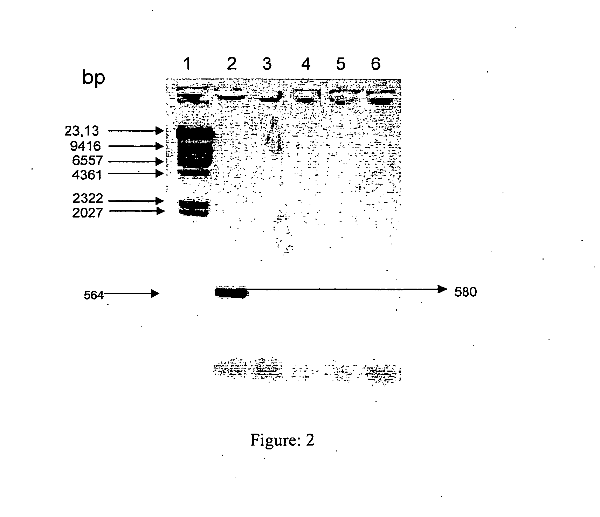

The amplified product was analyzed on agarose gel electrophoresis. The results obtained are shown in Figure 2. Lane 1 contains phage lambda DNA digested with *Hind* III marker. The sample in lane 2 contains DNA from the EE-1 elite event, while samples in lane 3 and 4 contain DNA from other transgenic brinjal plants that do not contain the EE-1 elite event. Lane 5 represents a non-transgenic brinjal control, and lane 6, the no DNA control sample. From the figure it is evident that the 580 bp fragment is amplified from the brinjal EE-1 elite event but not from other transgenic events and non-transgenic brinjal plants.

### Example 5

### Zygosity assay for brinjal EE-1 elite event

The genomic region around insertion site was characterized. The one end of the insertion site was analyzed the sequence and the sequence is as set forth in the SEQ ID NO: 8. Brinjal genomic DNA sequence from other flanking side of the T-DNA was analyzed and a primer was designed having nucleotide sequence as shown in SEQ ID NO: 15. This primer when used in combination with the primer having nucleotide sequence as set forth in SEQ ID NO: 11 and SEQ ID NO: 12, 580 base pairs fragment was obtained from the transgenic brinjal plant comprising EE-1 elite event due to amplification of the transgene specific allele and 330 base pairs fragment was obtained from non-transgenic brinjal plant due to amplification of non-transgenic allele band from SEQ ID NO: 11 and SEQ ID NO: 15.
MHTBJ-6: 5'-CCA TCT ACT AAC GGT GAT GGT -3' SEQ ID NO: 15

To identify the homozygous and heterozygous transgenic brinjal plant comprising the EE-1 elite event polymerase chain reaction was carried out using three primers having nucleotide sequence as shown in SEQ ID NO: 11, SEQ ID NO: 12 and SEQ ID NO: 15.

Genomic DNA from transgenic brinjal plants comprising the EE-1 elite event and non-transgenic brinjal plant was isolated using the method known in the art. Using this DNA as template as DNA PCR analysis was carried out. The PCR conditions for conducting zygosity PCR for EE-1 elite event is given below.

| **Reagents** | **Amount to be added** Make up to 25 µl |
|---|---|
| Nuclease-free water | |
| 10X reaction buffer (with MgCl₂) | 2.5 µl |
| 10mM dNTPs | 0.5 µl |
| Primer MHTBJ-6 (100 ng/µl) | 1.0 µl |
| Primer MHIP-2 (100 ng/µl) | 1.0 µl |
| Primer MHTBJ-2 (100 ng/µl) | 1.0 µl |
| Taq DNA polymerase (5 units/µl) | 0.5 µl |
| DNA template | 2.0 µl |

### Thermal Cycler program:

| **Temperature** | **Time** | **Cycles** |
|---|---|---|
| 95°C | 5 minutes | 1 |
| 94°C | 40 seconds | |
| 58°C | 40 seconds | 35 |
| 72°C | 1: 40minute | |
| 72°C | 5 minutes | 1 |
| 4°C | Hold | --- |

Polymerase chain reaction amplified a fragment of 580 base pairs from homozygous brinjal plant comprising the EE-1 elite event; two fragments of 580 and 330 base pairs size were obtained from the heterozygous brinjal plant comprising the EE-1 event and a fragment of 330 base pair size was obtained from the non transgenic brinjal plant.
SEQ ID NO: 16

ATGGACAACAACCCAAACATCAACGAATGCATTCCATACAACTGCTTGAGTAACCCA
GAAGTTGAAGTACTTGGTGGAGAACGCATTGAAACCGGTTACACTCCCATCGACATC
TCCTTGTCCTTGACACAGTTTCTGCTCAGCGAGTTCGTGCCAGGTGCTGGGTTCGTT
CTCGGACTAGTTGACATCATCTGGGGTATCTTTGGTCCATCTCAATGGGATGCATTC
CTGGTGCAAATTGAGCAGTTGATCAACCAGAGGATCGAAGAGTTCGCCAGGAACCAG

GCCATCTCTAGGTTGGAAGGATTGAGCAATCTCTACCAAATCTATGCAGAGAGCTTC
AGAGAGTGGGAAGCCGATCCTACTAACCCAGCTCTCCGCGAGGAAATGCGTATTCAA
TTCAACGACATGAACAGCGCCTTGACCACAGCTATCCCATTGTTCGCAGTCCAGAAC
TACCAAGTTCCTCTCTTGTCCGTGTACGTTCAAGCAGCTAATCTTCACCTCAGCGTG
CTTCGAGACGTTAGCGTGTTTGGGCAAAGGTGGGATTCGATGCTGCAACCATCAAT
AGCCGTTACAACGACCTTACTAGGCTGATTGGAAACTACACCGACCACGCTGTTCGT
TGGTACAACACTGGCTTGGAGCGTGTCTGGGGTCCTGATTCTAGAGATTGGATTAGA
TACAACCAGTTCAGGAGAGAATTGACCCTCACAGTTTTGGACATTGTGTCTCTCTTC
CCGAACTATGACTCCAGAACCTACCCTATCCGTACAGTGTCCCAACTTACCAGAGAA
ATCTATACTAACCCAGTTCTTGAGAACTTCGACGGTAGCTTCCGTGGTTCTGCCCAA
GGTATCGAAGGCTCCATCAGGAGCCCACACTTGATGGACATCTTGAACAGCATAACT
ATCTACACCGATGCTCACAGAGGAGAGTATTACTGGTCTGGACACCAGATCATGGCC
TCTCCAGTTGGATTCAGCGGGCCCGAGTTTACCTTTCCTCTCTATGGAACTATGGGA
AACGCCGCTCCACAACAACGTATCGTTGCTCAACTAGGTCAGGGTGTCTACAGAACC
TTGTCTTCCACCTTGTACAGAAGACCCTTCAATATCGGTATCAACAACCAGCAACTT
TCCGTTCTTGACGGAACAGAGTTCGCCTATGGAACCTCTTCTAACTTGCCATCCGCT
GTTTACAGAAAGAGCGGAACCGTTGATTCCTTGGACGAAATCCCACCACAGAACAAC
AATGTGCCACCCAGGCAAGGATTCTCCCACAGGTTGAGCCACGTGTCCATGTTCCGT
TCCGGATTCAGCAACAGTTCCGTGAGCATCATCAGAGCTCCTATGTTCTCTTGGATA
CACCGTAGTGCTGAGTTCAACAACATCATCGCATCCGATAGTATTACTCAAATCCCT
GCAGTGAAGGGAAACTTTCTCTTCAACGGTTCTGTCATTTCAGGACCAGGATTCACT
GGTGGAGACCTCGTTAGACTCAACAGCAGTGGAAATAACATTCAGAATAGAGGGTAT
ATTGAAGTTCCAATTCACTTCCCATCCACATCTACCAGATATAGAGTTCGTGTGAGG
TATGCTTCTGTGACCCCTATTCACCTCAACGTTAATTGGGGTAATTCATCCATCTTC
TCCAATACAGTTCCAGCTACAGCTACCTCCTTGGATAATCTCCAATCCAGCGATTTC
GGTTACTTTGAAAGTGCCAATGCTTTTACATCTTCACTCGGTAACATCGTGGGTGTT
AGAAACTTTAGTGGGACTGCAGGAGTGATTATCGACAGATTCGAGTTCATTCCAGTT
ACTGCAACACTCGAGGCTGAGTACAACCTTGAGAGAGCCCAGAAGGCTGTGAACGCC
CTCTTTACCTCCACCAATCAGCTTGGCTTGAAAACTAACGTTACTGACTATCACATT
GACCAAGTGTCCAACTTGGTCACCTACCTTAGCGATGAGTTCTGCCTCGACGAGAAG
CGTGAACTCTCCGAGAAAGTTAAACACGCCAAGCGTCTCAGCGACGAGAGGAATCTC
TTGCAAGACTCCAACTTCAAAGACATCAACAGGCAGCCAGAACGTGGTTGGGGTGGA
AGCACCGGGATCACCATCCAAGGAGGCGACGATGTGTTCAAGGAGAACTACGTCACC
CTCTCCGGAACTTTCGACGAGTGCTACCCTACCTACTTGTACCAGAAGATCGATGAG
TCCAAACTCAAAGCCTTCACCAGGTATCAACTTAGAGGCTACATCGAAGACAGCCAA
GACCTTGAAATCTACTCGATCAGGTACAATGCCAAGCACGAGACCGTGAATGTCCCA
GGTACTGGTTCCCTCTGGCCACTTTCTGCCCAATCTCCCATTGGGAAGTGTGGAGAG
CCTAACAGATGCGCTCCACACCTTGAGTGGAATCCTGACTTGGACTGCTCCTGCAGG
GATGGCGAGAAGTGTGCCCACCATTCTCATCACTTCTCCTTGGACATCGATGTGGGA
TGTACTGACCTGAATGAGGACCTCGGAGTCTGGGTCATCTTCAAGATCAAGACCCAA
GACGGACACGCAAGACTTGGCAACCTTGAGTTTCTCGAAGAGAAACCATTGGTCCGG
TGAAGCTCTCGCTCGTGTGAAGAGAGCAGAGAAGAAGTGGAGGGACAAACGTGAGAA
ACTCGAATGGGAAACTAACATCGTTTACAAGGAGGCCAAAGAGTCCGTGGATGCTTT

GTTCGTGAACTCCCAATATGATCAGTTGCAAGCCGACACCAACATCGCCATGATCCA
CGCCGCAGACAAACGTGTGCACAGCATTCGTGAGGCTTACTTGCCTGAGTTGTCCGT
GATCCCTGGTGTGAACGCTGCCATCTTCGAGGAACTTGAGGACGTATCTTTACCGC
ATTCTCCTTGTACGATGCCAGAAACGTCATCAAGAACGGTGACTTCAACAATGGCCT
CAGCTGCTGGAATGTGAAAGGTCATGTGGACGTGGAGGAACAGAACAATCAGCGTTC
CGTCCTGGTTGTGCCTGAGTGGGAAGCTGAAGTGTCCCAAGAGGTTAGAGTCTGTCC
AGGTAGAGGCTACATTCTCCGTGTGACCGCTTACAAGGAGGGATACGGTGAGGGTTG
CGTGACCATCCACGAGATCGAGAACAACACCGACGAGCTTAAGTTCTCCAACTGCGT
CGAGGAAGAAATCTATCCCAACAACACCGTTACTTGCAACGACTACACTGTGAATCA
GGAAGAGTACGGAGGTGCCTACACTAGCCGTAACAGAGGTTACAACGAAGCTCCTTC
CGTTCCTGCTGACTATGCCTCCGTGTACGAGGAGAAATCCTACACAGATGGCAGACG
TGAGAACCCTTGCGAGTTCAACAGAGGTTACAGGGACTACACACCACTTCCAGTTGG
CTATGTTACCAAGGAGCTTGAGTACTTTCCTGAGACCGACAAAGTGTGGATCGAGAT
CGGTGAAACCGAGGGAACCTTCATCGTGGACAGCGTGGAGCTTCTCTTGATGGAGGA
AAGATCTAGAGGCCTGAATTCGAGCTCGGTACCCGGGGATCCGTCCTTTGTCTTCA
ATTTTGAGGGCTTTTTACTGAATAAGTATGTAGTACTAAAATGTATGCTGTAATAGC
TCATAGTGAGCGAGGAAAGTATCGGGCTATTTAACTATGACTTGAGCTCCATCTATG
AATAAATAAATCAGCATATGATGCTTTTGTTTTGTGTACTTCAACTGTCTGCTTAGC
TAATTTGATATGGTTGGCACTTGGCACGTATAAATATGCTGAAGTAATTTACTCTGA
AGCTAAATAACTAGATTAGATGAGTGTATTATATACAAAAGGCATTAAATCAGATAC
ATCTTAGACAAATTGTCACGGTCTACCAGAAAAGAAATTGCATTTGTTTTTGGTCT
TTCAGACTGACAAGATCGATCTGAAGTCTAAACAATTCTAAGAGGTATCATGTAGCA
ATGTCCTGCCACAATATTGAATTGACCTGCAGGCATGCAAGCTGGATCAGATAGTC
GTTTCCCGCCTTCAGTTTAAACTATCAGTGTTTGTTACCAAAAGTGCTGTCAATAAA
CACTTAGAAAGGTGTTATCCAAATTTAAAAAGAAAAAAATAAATGTAAGATTTAAA
TTTCTAACTTCAAGAGATGCATTCAATTATCACCGCATTATATGATATTCCAAGAAT
ACCTGCCCGGGCGGCCGCTCGAGCCCTATA

### References

1. Cottage, A., Yang, A., Maunders, H., de Lacy, R.C. and Ramsay, N.A. (2001) Identification of DNA sequences flanking T-DNA insertions by PCR-walking. Plant Molecular Biology Reporter 19:321-327.
2. Fári, M., Nagy, I., Csányi, M., Mitykó, J. and Andrásfalvy A. (1995) Agrobacteriun mediated genetic transformation and plant regeneration via organogenesis and somatic embryogenesis from cotyledon leaves in eggplant (Solanum melongena L. cv. 'Kecskemeti lila'). Plant Cell Reports 15: 82-86.
3. Kumar, A. P., Mandaokar, A., Sreenivasu, K., Chakrabarti, S. K., Bisaria, S. and Sharma, R. P. (1998) Insect-resistant transgenic brinjal plants. Molecular Breeding 4:33-37.
4. Nicola, L.P., Valeria, B. and Leonardo, R. G. (1998) Regeneration of transgenic eggplants (Solanum melongena L.) for a cysteine proteinase inhibitor. Capsicum and Eggplant Newsletter, 17:92-95.
5. Rotino, G. L. and Gleddie, S. (1990) Transformation of Eggplant (Solanum melongena L.) using a binary Agrobacterium tumefaciens vector. Plant Cell Reports, 9:26-29.

## Claims

1. A method of detecting the presence of nucleic acid sequence of insect resistant brinjal plant EE-1 event comprising *cry1Ac* gene in a sample, wherein said method comprises:
a) contacting the sample with a first nucleotide sequence as set forth in the SEQ ID NO: 11, and a second nucleotide sequence selected from a group consisting of SEQ ID NO: 12 or SEQ ID NO: 13 or SEQ ID NO: 14.
b) performing a nucleic acid amplification reaction, thereby producing an amplified fragment; and
c) detecting said amplified fragment.

2. An isolated polynucleotide useful for detection of insect resistant brinjal plant EE-1 event comprising *cry1Ac* gene, wherein said polynucleotide comprises the polynucleotide sequence as set forth in SEQ ID NO: 8 or complement thereof.

3. An isolated polynucleotide useful for detection of insect resistant brinjal plant EE-1 event comprising *cry1Ac* gene, wherein said polynucleotide comprises the polynucleotide sequence as set forth in SEQ ID NO: 9 or complement thereof.

4. An isolated polynucleotide useful for detection of insect resistant brinjal plant EE-1 event comprising *cry1Ac* gene, wherein said polynucleotide comprises the polynucleotide sequence as set forth in SEQ ID NO: 10 or complement thereof.

5. A kit for detecting the presence of nucleic acid sequence of insect resistant brinjal plant EE-1 event comprising *cry1Ac* gene in a sample; wherein said kit comprises a first nucleotide having sequence as set forth in the SEQ ID NO: 11 and a second nucleotide sequence selected from a group consisting of SEQ ID NO: 12 or SEQ ID NO: 13 or SEQ ID NO: 14.

6. A synthetic oligonucleotide set comprising two oligonucleotides for detecting the presence of insect resistant brinjal plant EE-1 event comprising *cry1Ac* gene, wherein the first oligonucleotide comprises the nucleotide sequence as set forth in SEQ ID NO: 11 and the second oligonucleotide comprises the nucleotide sequence selected from a group consisting of SEQ ID NO: 12, SEQ ID NO: 13 and SEQ ID NO: 14.

## Patentansprüche

1. Ein Verfahren zur Detektion der Anwesenheit einer Nukleinsäuresequenz von Insekten-resistentem Auberginen-Pflanzen EE-1 Event umfassend *cryAc* Gen in einer Probe, wobei das Verfahren umfasst:
(a) in Kontakt bringen der Probe mit einer ersten Nukleotidsequenz, wie in der SEQ ID NO. 11 darstellt, und einer zweiten Nukleotidsequenz ausgewählt aus einer Gruppe bestehend aus SEQ ID NO. 12 oder SEQ ID NO. 13 oder SEQ ID NO. 14;
(b) Durchführen einer Nukleinsäure-Amplifizierungsreaktion, dabei ein modifiziertes Fragment produzierend; und
(c) Detektieren des amplifizierten Fragments.

2. Ein isoliertes Polynukleotid verwendbar für die Detektion von Insekten-resistentem Auberginen-Pflanzen EE-1 Event umfassend *cryAc* Gen, wobei das Polynukleotid die Polynukleotidsequenz, wie in SEQ ID NO. 8 dargestellt, oder ein Komplement davon umfasst.

3. Ein isoliertes Polynukleotid verwendbar für die Detektion von Insekten-resistentem Auberginen-Pflanzen EE-1 Event umfassend *cryAc* Gen, wobei das Polynukleotid die Polynukleotidsequenz, wie in SEQ ID NO. 9 dargestellt, oder ein Komplement davon umfasst.

4. Ein isoliertes Polynukleotid verwendbar für die Detektion von Insekten-resistentem Auberginen-Pflanzen EE-1 Event umfassend *cryAc* Gen, wobei das Polynukleotid die Polynukleotidsequenz, wie in SEQ ID NO. 10, dargestellt oder ein Komplement davon umfasst.

5. Ein Kit zur Detektion der Anwesenheit einer Nukleinsäuresequenz von Insekten-resistentem Auberginen-Pflanzen EE-1 Event umfassend *cryAc* Gen in einer Probe, wobei das Kit ein erstes Nukleotid, das die Sequenz, wie in der SEQ ID NO. 11 darstellt, aufweist, und eine zweite Nukleotidsequenz ausgewählt aus einer Gruppe bestehend aus SEQ ID NO. 12 oder SEQ ID NO. 13 oder SEQ ID NO. 14 umfasst.

6. Ein synthetisches Oligonukleotid-Set umfassend zwei Oligonukleotide zur Detektion der Anwesenheit von Insekten-resistentem Auberginen-Pflanzen EE-1 Event umfassend *cryAc* Gen, wobei das erste Oligonukleotid die Sequenz, wie in der SEQ ID NO. 11 darstellt, umfasst, und das zweite Oligonukleotid die Nukleotidsequenz ausgewählt aus einer Gruppe bestehend aus SEQ ID NO. 12 oder SEQ ID NO. 13 oder SEQ ID NO. 14 umfasst.

## Revendications

1. Procédé pour détecter la présence d'une séquence d'acide nucléique d'un événement EE-1 de plant d'aubergine résistant aux insectes, comprenant le gène *cry1Ac,* dans un échantillon, ledit procédé comprenant :
a) la mise en contact de l'échantillon avec une première séquence de nucléotides représentée dans la SEQ ID N°:11, et une seconde séquence de nucléotides choisie dans un groupe consistant en la SEQ ID N°:12, la SEQ ID N°:13 et la SEQ ID N°:14.
b) la conduite d'une réaction d'amplification d'acide nucléique, en produisant ainsi un fragment amplifié ; et
c) la détection dudit fragment amplifié.

2. Polynucléotide isolé utile pour la détection d'un événement EE-1 de plant d'aubergine résistant aux insectes, comprenant le gène *cry1Ac*, ledit polynucléotide comprenant la séquence polynucléotidique représentée dans la SEQ ID N°:8 ou son complément.

3. Polynucléotide isolé utile pour la détection d'un événement EE-1 de plant d'aubergine résistant aux insectes, comprenant le gène *cry1Ac,* ledit polynucléotide comprenant la séquence polynucléotidique représentée dans la SEQ ID N°:9 ou son complément.

4. Polynucléotide isolé utile pour la détection d'un événement EE-1 de plant d'aubergine résistant aux insectes, comprenant le gène *cry1Ac*, ledit polynucléotide comprenant la séquence polynucléotidique représentée dans la SEQ ID N°:10 ou son complément.

5. Kit pour la détection de la présence d'une séquence d'acide nucléique d'un événement EE-1 de plant d'aubergine résistant aux insectes, comprenant le gène *cry1Ac*, dans un échantillon ; ledit kit comprenant une première séquence de nucléotides représentée dans la SEQ ID N°:11 et une seconde séquence de nucléotides choisie dans un groupe consistant en les SEQ ID N°:12, la SEQ ID N°:13 et la SEQ ID N°:14.

6. Ensemble d'oligonucléotides synthétiques comprenant deux oligonucléotides pour la détection de la présence d'un événement EE-1 de plant d'aubergine résistant aux insectes, comprenant le gène *cry1Ac*, dans lequel le premier oligonucléotide comprend la séquence de nucléotides représentée dans la SEQ ID N°:11 et le second oligonucléotide comprend la séquence de nucléotides choisie dans un groupe consistant en la SEQ ID N°:12, la SEQ ID N°:13 et la SEQ ID N°:14.
